# EUROPEAN PATENT APPLICATION

(11) **EP 4 403 530 A1**
(43) Date of publication of application: **24.07.2024**
(21) Application number: 23219574.3
(22) Date of filing: 22.12.2023
(51) Int. Cl.: C03C 3/087, A61K 6/836, C03C 3/089, C03C 3/091, C03C 4/00, C03C 8/02, C03C 8/04, C03C 8/16, C04B 41/00

(54) **DENTAL PORCELAIN PASTE CONTAINING INORGANIC SALT**

(30) Priority: 27.12.2022 JP 2022210988
(71) Applicant: Shofu Inc., Kyoto-shi, Kyoto 605-0983 (JP)
(72) Inventor: TANAKA, Yosuke, Kyoto, 605-0983 (JP); GOTO, Masanori, Kyoto, 605-0983 (JP)
(74) Representative: Eisenführ Speiser

(57) **Abstract**

[Problem]

To provide a dental porcelain paste that can maintain a constant paste property even during long-term storage, and that hardly generates carbonization and bubbles during firing even in the case of containing organic components.

[Solution]

To provide a dental porcelain paste for preparing a dental prosthesis device, comprising (a) base material glass having an average particle diameter D50 of 1 to 20 µm, (b) hydrophobized fine particle silica having an average primary particle diameter of 1 to 50 nm, (c) organic solvent having a boiling point of 100 to 300°C, and (d) inorganic salt.

## Description

### BACKGROUND OF THE INVENTION

### Field of the Invention

The present invention relates to a dental porcelain paste that is used in a dental prosthesis device such as an artificial tooth, maintains a constant paste property even during long-term storage, and has excellent operability.

### Description of the Related Art

As materials for preparing a dental prosthesis device such as an inlay, a crown and a bridge, there is a growing demand for core materials that are less allergenic and have excellent strength and aesthetic property, such as oxide ceramics such as alumina and zirconia, and glass ceramics such as feldspar-base glass and lithium silicate glass. Particularly in recent years, with the development of CAD/CAM technology, an easy technique for securing the shape of a dental prosthesis device using single material are becoming mainstream. However, there is a problem in oxide ceramics that transparency is still low despite advances in multilayer technology, and in glass ceramics that it is difficult to reproduce the partial color tone unique to a natural tooth although transparency is high. Therefore, under the present situation, material properties of single material are not sufficient to ensure aesthetic property. In order to impart a dental prosthesis device with aesthetic property equivalent to that of a natural tooth, it is necessary to use a combination material that complements luster, transparency and coloring.

Glass material, glass ceramic material, oxide ceramic material and the like are generally selected as materials that impart luster, transparency and coloring to the core materials of oxide ceramics and glass ceramics. These materials are usually used by kneading a powdered base material glass with a liquid component that disappears during baking to form a paste. In this case, it is preferable to make in a paste form (slurry) having suitable viscous for application in advance so as not to be influenced by the skill of the operator. As an example of the kneading liquid used in this technique, Japanese Unexamined Patent Application Publication No. 2017-193492 discloses an organic solvent that contains 5 wt.% or more of an ester compound not containing an aromatic ring and does not contain a polymerizable monomer. However, in the paste prepared by simply mixing these kneaded liquids with the base material glass, changes during long-term storage are significant, and sedimentation of the paste, liquid separation and a decrease in consistency (viscosity) due to blending of the glass and liquid over time and the like occur. Such changes over time deteriorate work efficiency and make it difficult to maintain a constant workability in dental technique work that involves precise work.

Such changes over time are caused by a change in the state of particle existence in the paste, and specifically, agglomeration of particles, sedimentation of particles and aggregates and the like occur. In order to suppress this change, there is a method of separately adding an organic polymer dispersant to disperse particles in the paste to maintain constant paste properties even during long-term storage. Japanese Unexamined Patent Application Publication No. 2001-079019 discloses a dental paste porcelain material that is hardly dried and solidified during use.

The feature is that the dental porcelain paste comprises a mixture in a paste-like state of from 7 to 45 parts by weight of an organic solvent which has a viscosity of 50,000 to 1,500,000 cps and a porcelain powder as a remainder, with a total amount being 100 parts by weight. However, in this case, carbonization and bubble generation may occur due to the influence of the organic polymer component during firing.

While the technique of preparing in a paste state in advance is useful, there is a problem that it is not possible to maintain a certain paste property during long-term storage, or that it is necessary to add stabilizing components that have the risk of adversely affecting luster, transparency and color tone in order to maintain a certain paste property.

### SUMMARY OF THE INVENTION

### Technical Problem

An object of the present invention is to provide a dental porcelain paste that can maintain a constant paste property even during long-term storage, and that hardly generates carbonization and bubbles during firing even in the case of containing organic components.

### Solution to Problem

The dental porcelain paste for preparing a dental prosthesis device of the present invention comprises (a) base material glass having an average particle diameter D50 of 1 to 20 µm, (b) hydrophobized fine particle silica having an average primary particle diameter of 1 to 50 nm, (c) organic solvent having a boiling point of 100 to 300°C, and (d) inorganic salt.

The "average particle diameter D50" in the present invention means a median diameter representing the average value of particles. The average particle diameter of the base material glass can be determined by measurement methods, for example, using a laser diffraction scattering method, a dynamic light scattering method, a centrifugal sedimentation method, an electrical sensing zone method, a sieving method, a photon correlation spectroscopy method or the like. The (a) base material glass of the present invention may have an average particle diameter D50 of 1 to 20 µm in any measurement method, and in particular, may have an average particle diameter D50 of 1 to 20 µm measured by a laser diffraction scattering method.

The "average primary particle diameter" in the present invention means an average value of all particle diameters in a state where they are not aggregated. The average primary particle diameter of the hydrophobized fine particle silica can be calculated from, for example, the specific surface area obtained by measurement using a gas absorption method, a mercury intrusion method, a gas permeation method, a bubble point method, or the like. The (b) hydrophobized fine particle silica of the present invention may have an average primary particle diameter of 1 to 50 nm in any measurement method, and in particular, may have an average primary particle diameter of 1 to 50 nm measured by a gas absorption method.

### Advantageous Effects of Invention

The dental porcelain paste of the present invention can maintain a constant paste property even during long-term storage, and can suppress black discoloration due to carbonization and cloudiness due to bubbles during firing even in the case of containing organic components.

### DETAILED DESCRIPTION OF THE PREFERRED EMBODIMENTS

The dental porcelain paste of the present invention is a dental porcelain paste for preparing a dental prosthesis device, comprising (a) base material glass having an average particle diameter D50 of 1 to 20 µm, (b) hydrophobized fine particle silica having an average primary particle diameter of 1 to 50 nm, (c) organic solvent having a boiling point of 100 to 300°C, and (d) inorganic salt.

The dental porcelain paste of the present invention may comprise, with respect to 100 parts by mass of the (a) base material glass, 1 to 15 parts by mass of the (b) hydrophobized fine particle silica, 15 to 90 parts by mass of the (c) organic solvent, and 0.05 to 1.0 parts by mass of the (d) inorganic salt.

The dental porcelain paste of the present invention may further comprise (e) coloring material and/or (f) fluorescent material which are inorganic materials.

In the dental porcelain paste of the present invention, a maximum particle diameter D100 of the (a) base material glass may be 200 µm or less.

The dental porcelain paste is used, for example, by applying or building up on a core material and firing it one or more times. By this, it is possible to obtain the shape, luster, and color tone of the desired dental prosthesis device. The "core material" in the present invention may be, for example, oxide ceramics such as alumina and zirconia, and glass or glass ceramics such as feldspar-base glass and lithium silicate glass.

As the temperature for the firing a dental porcelain paste (hereinafter referred to as firing temperature), the temperature range of 650 to 1000°C is generally selected. When the firing is performed at a temperature lower than 650 °C, there is a case where the organic solvent is not fired sufficiently and is carbonized, therefore it is impossible to obtain the desired color tone. When the firing is performed at a temperature higher than 1000 °C, there is a case where deformation or sagging occurs on the core material itself or on the application surface of the porcelain paste applied to the dental prosthesis device.

It is not preferable that the appropriate firing temperature for firing the dental porcelain paste (hereinafter referred to as the "appropriate firing temperature") deviates significantly from the appropriate firing temperature of the (a) base material glass constituting the dental porcelain paste. That is, when the dental porcelain paste is fired at the same temperature as the appropriate firing temperature of the base material glass, it is preferable that the application surface of the dental porcelain paste after firing is not insufficiently fired (loss of gloss). The reason for this is to prevent the original characteristics of the base material glass, such as firing property and physical property, from being lost by making it into a paste. In the present invention, the appropriate firing temperature of the dental porcelain paste means a temperature that can impart luster to the surface of the dental porcelain paste layer after firing, and may be a temperature range. Further, in the present invention, the appropriate firing temperature of the base material glass means a temperature that can impart luster to the surface of the base material glass layer after firing, and may be a temperature range.

In the present invention, the appropriate firing temperature of the (a) base material glass may be in the range of 650 to 1000 °C. Although the range of the appropriate firing temperature is not particularly limited, the base material glass having an appropriate firing temperature of less than 650 °C cannot be used because it is difficult to prepare from the viewpoint of aesthetic properties and biosafety. The base material glass having a appropriate firing temperature exceeding 1000 °C is not preferable because there is a case where deformation or sagging occurs on the core material itself or on the application surface of the porcelain paste applied to the dental prosthesis device.

The dental porcelain paste may be fired only once. Further, the dental porcelain paste may be fired multiple times, such as by firing after building up the dental porcelain paste, and then by firing after further building up the dental porcelain paste. Such firing can be performed by using a dental technique porcelain furnace capable of vacuum firing at a heating rate of 10 to 100 °C/min and a firing temperature range of 100 to 1200 °C.

The (a) base material glass is a glass component that serves as a base material for a dental prosthesis device obtained by firing and is melted by firing to fix and bond to the core material. In the present invention, the (a) base material glass can be used not only singly but also in appropriate combinations of two or more, as long as it is fixed to the core material by firing.

The average particle diameter D50 of the (a) base material glass is 1 to 20 µm, and may be 2 to 15 µm, and may be 4 to 10 µm. When the average particle diameter D50 is smaller than 1 µm, the glass particles may aggregate with each other in forming a paste, and the consistency may be changed. When the average particle diameter is larger than 20 µm, there is a case where the base material tends to settle in forming a paste.

The "average particle diameter D50" in the present invention means the median diameter, that is, D50/µm. The value of D50/µm being X means that 50% of the particles in the volume to be analyzed have a particle size of less than X µm. The "average particle diameter D50" is different from other averaging methods (arithmetical mean of number, length, volume, area and the like) and mode diameter (most frequent particle diameter).

The maximum particle size D100/µm of the base material glass may be 200 µm or less, 175 µm or less, and 100 µm or less. The "maximum particle diameter D100" in the present invention means the maximum value of the particle diameter in the volume to be analyzed. In the case of containing particles larger than 200 µm, there is a tendency that the operability is poor and the application surface after firing is not uniform. Furthermore, there is a tendency that the base material settles in forming a paste.

The preparation method of the base material glass may be performed without limitation by the common preparation device and method of the glass composition held by a person skilled in the art. In one of the common preparation method, various inorganic compounds are compounded so as to obtain a targeted glass composition to melt at 1300 to 1500 °C using a glass melting furnace. The melt is charged into water to be quenched (quenching) to prepare a glass frit. In order to use the base material glass obtained by this method as a dental porcelain paste, it is necessary to powder the glass frit in order to adjust the particle size. Examples of methods for powdering include a method of crushing the glass frit described above with a crusher such as a rotary ball mill, a vibration ball mill, a planetary mill, a jet mill, a bead mill, a roll crusher, a jaw crusher and the like. Further, in order to uniformly adjust the particle size of the base material glass, in addition to the pulverization of the base material glass, classification may be performed using a sieve, an elutriation and the like as necessary.

The softening point (Ts) of the (a) base material glass is preferably within a range of 450 °C to 650 °C. By using the base material glass having a softening point (Ts) within the range of 450 °C to 650 °C, it is possible to carry out firing at 650 °C to 850 °C and it is possible to apply to a core material which is prepared from a lithium disilicate base glass ceramics which includes a risk of deformation in firing more than 850 °C.

The (a) base material glass may be compounded in an appropriate amount so as to obtain a desired paste property, but when the compounding amount of the (a) base material glass is too small (the compounding amount other than the (a) base material glass is too large), there is a tendency that luster and transparency derived from the (a) base material glass is insufficient and it is difficult to maintain paste property. When the compounding amount of the (a) base material glass is too large, there is a tendency that it is difficult to form a paste.

The (a) base material glass used in the present invention is not particularly limited as long as it can be used in a dental porcelain and has an average particle diameter D50 of 1 to 20 µm. In addition, it may be a glass ceramics containing crystals in glass. By including crystals, it is expected to adjust the thermal expansion coefficient of the dental porcelain paste and to improve the mechanical property.

Examples of the (a) base material glass include glass and crystallized glass which contain SiOz as a main component (component having the largest content). Such glass may contain Al₂O₃, B₂O₃, ZnO, KzO, NazO, LizO, ZrO₂, CaO, MgO and the like, in addition to SiO₂. Specific examples include amorphous type potassium aluminosilicate glass, amorphous type potassium borosilicate glass, crystal type potassium aluminosilicate glass, crystal type fluoroapatite glass and crystal type lithium silicate glass.

The (a) base material glass is the main component (component having the largest content) of the dental porcelain paste of the present invention, and is a component that determines the material characteristics and physical properties of the dental porcelain paste itself. Therefore, the elution amount, bending strength, and thermal expansion coefficient of the (a) base material glass used in the present invention can be made suitable for use as a dental porcelain material.

The (a) base material glass used in the present invention may have, for example, an elution amount of 50 µg/cm² or less, 35 µg/cm² or less, and 20 µg/cm² or less according to "Dentistry-Ceramic materials" of ISO6872:2015/Amd.1:2018. By setting the elution amount to 50 µg/cm² or less, the dental porcelain paste may meet the requirements of the ISO standard.

The (a) base material glass used in the present invention may have, for example, a bending strength of 50 MPa or more, 80 MPa or more, and 100 MPa or more according to "Dentistry-Ceramic materials" of ISO6872:2015/Amd.1:2018. By setting the bending strength to 50 MPa or more, the dental porcelain paste may meet the requirements of the ISO standard.

The (a) base material glass used in the present invention may have, for example, a thermal expansion coefficient of 7.0 to 14.0 × 10⁻⁶K⁻¹, and 7.5 to 11.0 × 10⁻⁶K⁻¹ according to "Dentistry-Ceramic materials" of ISO6872:2015/Amd.1:2018. The dental porcelain paste of the present invention is used, for example, for the purpose of fixing to the upper part of a core material made of oxide ceramics or glass ceramics. Because the thermal expansion coefficient of these core materials are approximately 10.0 to 11.0 × 10⁻⁶K⁻¹, it is possible to suppress the occurrence of cracks during the preparation of dental prosthesis devices, by setting the thermal expansion coefficients of the base material glass and the dental porcelain paste to a range slightly lower the range of core materials.

The average primary particle diameter of the (b) hydrophobized fine particle silica is 1 to 50 nm, may be 5 to 45 nm, and may be 7 to 40 nm. When the average primary particle diameter of the (b) hydrophobic fine particle silica is larger than 50 nm, there is a case where the paste consistency is changed. Hydrophobized fine particle silica having an average primary diameter particle of less than 1 nm cannot be used because it is difficult to prepare the fine particle silica itself.

The (b) hydrophobized fine particle silica is hydrophobized by a surface treatment with a surface treatment material. Examples of the surface treatment material include silane coupling materials. Silane coupling materials are not limited in particular, but specific examples include methyl trimethoxy silane, dimethyl dimethoxy silane, phenyl trimethoxy silane, diphenyl dimethoxy silane, methyl triethoxy silane, dimethyl diethoxy silane, phenyl triethoxy silane, diphenyl diethoxy silane, isobutyl trimethoxy silane, vinyl trimethoxy silane, vinyl triethoxy silane, vinyl tris (2-methoxyethoxy) silane, 3,3,3-trifluoro propyl trimethoxy silane, methyl-3,3,3-trifluoro propyl dimethoxy silane, 2-(3,4-epoxy cyclohexyl) ethyl trimethoxy silane, hexamethyl disilazane, 3-glycidoxypropyl trimethoxy silane, 3-glycidoxypropyl methyl diethoxy silane, 3-glycidoxypropyl triethoxy silane, 3-methacryloxy propyl methyl dimethoxy silane, polydimethyl siloxane, 3-methacryloxy propyl methyl diethoxy silane, N-2 (aminoethyl) 3-aminopropyl methyl dimethoxy silane, N-2 (aminoethyl) 3-aminopropyl trimethoxy silane, N-2 (aminoethyl) 3-aminopropyl triethoxy silane, 3-aminopropyl trimethoxy silane, 3-aminopropyl triethoxy silane, N-phenyl-3-aminopropyl trimethoxy silane, 3-mercaptopropyl trimethoxy silane, trimethyl silanol, methyl trichloro silane, methyl dichloro silane, dimethyl dichloro silane, trimethyl chloro silane, phenyl trichloro silane, diphenyl dichloro silane, vinyl trichloro silane, trimethyl bromo silane, diethyl silane, vinyl triacetoxy silane, co-(meth)acryloxyalkyl trimethoxy silane (carbon number between (meth)acryloxy group and silicon atom: 3-12, example: 3-methacryloxy propyl trimethoxy silane, etc.), co-(meth)acryloxy alkyl triethoxy silane (carbon number between (meth)acryloxy group and silicon atom: 3-12, example: 3-methacryloxy propyl triethoxy silane, etc.) and the like. Examples of the surface treatment method include a method of contacting the silane coupling material with the fine particle silica. Specific examples include a method comprising putting the fine particle silica into a heated reactor, and pneumatically dispatching the silane coupling material in the reactor in parallel by an inert gas such as nitrogen at a ratio of 0.01 to 0.5 kg per 1 kg of silica. In the present invention, silica other than the hydrophobized fine particle silica, for example, hydrophilic silica may be contained as long as the effect of the present invention is not affected. Specifically, in the present invention, the proportion of silica other than the hydrophobized fine particle silica may be 0 wt.% or more and 50 wt.% or less of the total amount of silica, and may be 0 wt.% or more and 20 wt.% or less, and 0 wt.% or more and 10 wt.% or less. Further, in the present invention, no silica other than the hydrophobized fine particle silica may be contained.

The (b) hydrophobized fine particle silica may be compounded in an appropriate amount according to a desired paste property, and the compounding amount may be 1 to 15 parts by mass, and 1.5 to 6.0 parts by mass based on 100 parts by mass of the (a) base material glass. When the compounding amount of the (b) hydrophobized fine particle silica is too small, there is a case where the paste consistency is changed. When the compounding amount of the (b) hydrophobized fine particle silica is too large, there is a case where it becomes difficult to fire a dental porcelain paste, and the gloss disappears due to insufficient firing in the case of firing the dental porcelain paste at the appropriate firing temperature of the base material glass. Further, there is a case where it is difficult to uniformly apply on a surface.

The (b) hydrophobized fine particle silica used in the present invention is not particularly limited as long as it has an average primary particle diameter of 1 to 50 nm, but specific examples include fumed silica, precipitated silica, colloidal anhydrous silica, silica gel, Syloid, Aerosil and the like. Further, the (b) hydrophobized fine particle silica can be used not only singly but also in appropriate combinations of two or more.

As the (c) organic solvent used in the present invention, an organic solvent having a boiling point of 100 to 300 °C is used. When the boiling point of the organic solvent is less than 100 °C, the organic solvent volatilizes even at room temperature and therefore operability during dental technique work deteriorates and it is difficult to maintain paste property for long-term. When the boiling point of the organic solvent exceeds 300 °C, it cause unburnt residue during firing, causing carbonization and bubbles.

In the present invention, the compounding amount of the (c) organic solvent may 15 to 90 parts by mass, and 30 to 60 parts by mass based on 100 parts by mass of the base material glass. When the compounding amount of the organic solvent is too small, it may be difficult to form a paste. When the compounding amount of the organic solvent is too large, there is a tendency that the consistency change and precipitation over time easily occur.

The (c) organic solvent used in the present invention is not particularly limited as long as it has a boiling point of 100 to 300 °C, but specific examples include ester base solvents such as dimethyl phthalate and diethyl phthalate; polyhydric alcohol base solvents such as 1,2-butanediol, 1,3-butanediol, 1,4-butanediol, 1,5-pentanediol, glycerin, diethylene glycol, triethylene glycol, propylene glycol and dipropylene glycol; polyhydric alcohol monoether base solvents such as ethylene glycol monomethyl ether, ethylene glycol monoethyl ether, propylene glycol monomethyl ether, propylene glycol monoethyl ether, ethylene glycol monobutyl ether, diethylene glycol monomethyl ether, diethylene glycol monoethyl ether, propylene glycol monopropyl ether, tripropylene glycol monomethyl ether, diethylene glycol monobutyl ether and triethylene glycol monomethyl ether; and aromatic alcohol solvents such as 2-phenoxy ethanol and benzyl alcohol. Among these organic solvents, polyhydric alcohol base solvents, polyhydric alcohol monoether base solvents and aromatic alcohol solvents may be used and 1,3-butanediol, propylene glycol and 2-phenoxy ethanol may be used. These organic solvents (c) can be used not only singly but also in appropriate combinations of two or more. When two or more kinds of organic solvents are used in combination, the total value obtained by multiplying the boiling points of the respective organic solvents to be used by the addition ratio is used as the boiling point of the organic solvents (c).

Furthermore, the (c) organic solvent used in the present invention may be an alcohol having a hydroxy group. The reason for this is to improve the compatibility with the base material glass having a hydroxyl group to improve the applicability.

The dental porcelain paste of the present invention may not contain water, except the case to be adsorbed to the base material glass or particles such as particulate silica. The dental porcelain paste of the present invention may not contain water as a solvent.

The dental porcelain paste of the present invention may not contain an organic polymeric solvent. The "organic polymer" in the present invention refers to an organic component having a molecular weight of 200 or more among components that are separately added for purposes other than surface treatment. Such organic component has poor volatility and there is a possibility that it remains during the firing step. The dental porcelain paste of the present invention may not contain any solvent other than the (c) organic solvent.

The dental porcelain paste of the present invention may contain an additive that stabilizes the particle state. Examples of the additive include dispersants, surfactants, and pH adjusters. However, in the case of using as a dental porcelain, when it remains during the firing process, it greatly affect the aesthetic properties. Therefore, the additive used in the present invention may be an inorganic salt that dissolves in an organic solvent and leaves little residue after incineration.

The (d) inorganic salt may be volatilized during the process of firing the dental porcelain paste, or may remain as a metal oxide similar to the constituent components of the (a) base material glass.

The (d) inorganic salt used in the present invention is not particularly limited, but may have solubility in the (c) organic solvents. Specific examples include a chloride, a nitrate, a sulfate, a carbonate and/or a hydrate thereof, which includes an element selected from Al, Zn, K, Na, Li, Ca and Mg. Further specific examples include aluminum chloride, aluminum nitrate, zinc chloride, zinc nitrate, potassium chloride, potassium sulfate, sodium chloride, sodium sulfate, lithium chloride, lithium nitrate, calcium chloride, calcium nitrate, calcium sulfate, magnesium nitrate, magnesium sulfate, and/or hydrates thereof. Further, these (d) inorganic salts can be used alone or in an appropriate combination of two or more.

In the present invention, "solubility in organic solvents" refers to the property that 1.0 parts by mass or less of an inorganic salt is dissolved in 15 parts by mass of an organic solvent. This can be determined by a general solubility measurement method. For example, when 1.0 parts by mass of an inorganic salt is added to 15 parts by mass of an organic solvent, if no undissolved part of the inorganic salt remains in the organic solvent, it may be determined that the inorganic salt has solubility in organic solvents. Further, dissolution in the organic solvent does not need to occur instantaneously, and may be achieved by external force such as ultrasonic vibration, physical pulverization, or heating.

In the present invention, the compounding amount of the (d) inorganic salt may be 0.05 to 1.0 parts by mass, and 0.1 to 0.5 parts by mass with respect to 100 parts by mass of the (a) base material glass. When the compounding amount of the (d) inorganic salt is too small, there is a tendency that the consistency change over time easily occurs. When the compounding amount of the (d) inorganic salt is too large, there is a case where dilatancy property may be strongly expressed to affect operability.

The dental porcelain paste of the present invention needs to comprise all of the (a) base material glass, the (b) hydrophobized fine particle silica, the (c) organic solvent, and the (d) inorganic salt. Furthermore, the dental porcelain paste of the present invention may comprise, with respect to 100 parts by mass of the (a) base material glass, 1 to 15 parts by mass of the (b) hydrophobized fine particle, and 0.05 to 1.0 parts by mass of the (d) inorganic salt, in order to suppress a change over time in the consistency of the paste. The dental porcelain paste of the present invention may comprise, with respect to 100 parts by mass of the (a) base material glass, 1.5 to 6.0 parts by mass of the (b) hydrophobized fine particle silica, and 0.1 to 0.5 parts by mass of the (d) inorganic salt. The dental porcelain paste of the present invention may comprise, with respect to 100 parts by mass of the (a) base material glass, 1 to 15 parts by mass of the (b) hydrophobized fine particle silica, 15 to 90 parts by mass of the (c) organic solvent, and 0.05 to 1.0 parts by mass of the (d) inorganic salt. The dental porcelain paste of the present invention may comprise, with respect to 100 parts by mass of the (a) base material glass, 1.5 to 6.0 parts by mass of the (b) hydrophobized fine particle silica, 30.0 to 60.0 parts by mass of the (c) organic solvent, and 0.1 to 0.50 parts by mass of the (d) inorganic salt.

The dental porcelain paste of the present invention may contain (e) coloring material and/or (f) fluorescent material. The coloring material (e) is, for example, an inorganic material, and those commonly used in the dental materials can be used. Specific examples include a coloring material prepared by mixing a plurality of metal oxides such as SiO₂, Al₂O₃, CaO, TiO₂, SnO, Cr₂O₃, MnO, Sb₂O₃, VzOs, ZnO, Fe₂O₃, W₂O₃, Co₂O₃ and ZrO₂, and firing. The compounding amount thereof is preferably within a range of 0.05 to 40 wt.% in the case that the total amount of the dental porcelain paste is 100.0 wt.%.

The (f) fluorescent material is, for example, an inorganic material, and those commonly used in the dental materials can be used. The compounding amount thereof is preferably within a range of 0.1 to 5.0 wt.% in the case that the total amount of the dental porcelain paste is 100.0 wt.%. The (e) colorant and/or the (f) fluorescent material may be an inorganic material. In the present invention, incinerable organic dyes may not be contained. The reason for this is that, like organic polymers, it remains during the firing stage and causes bubbles.

The preparation of the dental porcelain paste of the present invention can be performed without limitation by the common preparation method of the paste composition held by a person skilled in the art. In one of the common preparation method, the paste is prepared by compounding a base material glass, a hydrophobized fine particle silica, an organic solvent, an inorganic salt, a coloring material and a fluorescent material so that a target paste composition is obtained, and mixing with a device comprising a stirring and/or defoaming function. It is possible to perform the mixing by a known kneading method using a rotary mixer equipped with stirring blades, a rotation-revolution mixer, a crusher, a roll mill, a ball mill, a kneader and the like, and these methods can be used alone or in combination.

### [Examples]

Hereinafter, the present invention will be described in detail with reference to Examples and Comparative Examples. However, the present invention is not limited to these Examples.

Various evaluation methods for the average particle diameter D50 and the maximum particle diameter D100 of the base material glass, presence or absence of crystals, the appropriate firing temperature, the average primary particle diameter of the hydrophobized fine particle silica, and the dental porcelain paste in Examples and Comparative Examples are described below.

### [Measuring method of average particle diameter D50 and maximum particle diameter D100 of base material glass]

The average particle diameter D50 and the maximum particle diameter D100 of the base material glass can be determined by measurement using a laser diffraction scattering method, a dynamic light scattering method, a centrifugal sedimentation method, an electrical sensing zone method, a sieving method, a photon correlation spectroscopy method or the like. The particle diameters of the glass powders of Examples and Comparative Examples were measured by a laser diffraction scattering method. Specifically, the particle diameters were measured with a laser diffraction type particle size distribution measuring device Microtrac MT-3000II (manufactured by MicrotracBEL Corp).

### [Measuring method of presence or absence of crystals]

The presence or absence of crystals in the base material glass can be confirmed by measurement using an X-ray diffraction device. Specifically, the measurement was performed using the X-ray diffraction device Multiflex (manufactured by Rigaku Corp.) at a scanning range of 10 to 70° and a scanning speed of 2.0°/min.

### [Measuring method of appropriate firing temperature of base material glass]

The base material glass was kneaded with 1,3-butanediol to prepare a paste. The paste was applied to a 10.0 × 10.0 mm surface of a zirconia plate (10.0 × 10.0 × 2.0 mm) to a thickness of 0.2 mm using a dental technician brush. Vacuum firing was performed using the dental technique porcelain furnace "Esthemat Slim" (manufactured by SHOFU INC.). The lowest temperature at which gloss was exhibited on the application surface of the fired product was defined as the appropriate firing temperature of the base material glass. Table 2 shows the appropriate firing temperature of the base material glass.

### [Measuring method of softening point and thermal expansion coefficient of base material glass]

Each base material glass was kneaded with distilled water to prepare a kneaded material. The kneaded material was filled in a stick type mold made of the silicon (6 × 6 × 25 mm) and was subjected to condensation and water absorption repeatedly to prepare a molded body. The molded body was taken out from the silicon mold and was fired twice including one time vacuum firing and one time atmospheric firing by the dental technique porcelain furnace "Esthemat Slim" (manufactured by SHOFU INC.). Sample was prepared by polishing both ends of the prepared twice calcined product to prepare parallel faces and adjusting the size to 5 × 5 × 20 mm. Thermal expansion coefficient and softening point of the sample were measured by the thermal expansion meter "TM8140C" (manufactured by Rigaku Holdings Corporation) according to the procedure of ISO6872:2015 /Amd.1:2018 "Dentistry-Ceramic materials".

### [Measuring method of elution amount of base material glass]

Each base material glass was kneaded with distilled water to prepare a kneaded material. The kneaded material was filled in a disc type mold made of the silicon (ϕ12 mm × 2 mm) and was subjected to condensation and water absorption repeatedly to prepare a molded body. Ten fired products were prepared by taking out the molded body from the silicon mold and vacuum firing by the dental technique porcelain furnace "Esthemat Slim" (manufactured by SHOFU INC.). After surface polish of both surfaces of the fired products, second calcination (atmospheric calcination) was performed. The test specimen was tested according to the procedure of ISO6872:2015 /Amd.1:2018 "Dentistry-Ceramic materials".

### [Measuring method of bending strength of base material glass]

Each base material glass was kneaded with distilled water to prepare a kneaded material. The kneaded material was filled in a stick type mold made of the silicon (3 × 6 × 25 mm) and was subjected to condensation and water absorption repeatedly to prepare a molded body. Ten fired products were prepared by taking out the molded body from the silicon mold and vacuum calcination by the dental technique porcelain furnace "Esthemat Slim" (manufactured by SHOFU INC.). The entire surface of the prepared fired product was polished to make parallel surfaces, and the sample adjusted to a size of 1.2 × 4 × 20 mm was used as a test specimen. The bending strength was measured using a universal testing machine (manufactured by Shimadzu Corporation) according to the procedure of ISO6872:2015 /Amd.1:2018 "Dentistry-Ceramic materials".

### [Measurement method of average primary particle diameter of hydrophobized fine particle silica]

The average primary particle diameter of the hydrophobized fine particle silica can be calculated from the specific surface area obtained by measurement using a gas absorption method, a mercury intrusion method, a gas permeation method, a bubble point method, or the like. The primary particle diameters of the hydrophobized fine particle silicas of the examples and comparative examples were measured by a gas absorption method. Specifically, the primary particle diameters were measured with micromeritics automatic surface area and porosimetry Analyzer, TriStar II 3020 (manufactured by Shimadzu Corporation).

### [Evaluation 1: Firing property of base material glass at appropriate firing temperature (black discoloration)]

Dental porcelain paste was applied to a 10.0 × 10.0 mm surface of a zirconia plate (10.0 × 10.0 × 2.0 mm) to a thickness of 0.2 mm using a dental technician brush. Vacuum firing was performed at the appropriate firing temperature (minimum temperature) of the compounded base material glass using the dental technique porcelain furnace "Esthemat Slim" (manufactured by SHOFU INC.). The firing property (black discoloration) of the application surface of the fired product prepared by this method was evaluated. The evaluation criteria are shown below.
A: A state in which glass layer has transparent. In the case of Example 4 which has a color tone, the color is sufficiently exhibited developed.
B: A state in which black discoloration occurs due to carbonization and a decrease in brightness is observed in the glass layer.

### [Evaluation 2: Bubbles in paste]

One location on the application surface of the fired product prepared in Evaluation 1 was observed in a 100 µm × 100 µm field of view and evaluated. The evaluation criteria are shown below.
A: Less than 10 bubbles of 5 µm or more were observed.
B: 10 or more bubbles of 5 µm or more were observed.

### [Evaluation 3: Consistency and change amount of paste]

In this evaluation, the paste of 5 g which was filled into an 8 mL cream container and stored at 23 °C for 1 or 14 days or at 50 °C for 14 days after preparing the paste was used.

The paste was left in a thermostatic room at 23 °C before one hour of conducting the evaluation. The paste within a range of 0.3 ± 0.03 g was measured and the measured paste was taken out and placed on a glass plate. One more glass plate was placed on the paste, and furthermore 20 g of a weight was placed thereon. After 30 seconds from placing the weight, the weight was removed. The paste spread in a circular shape was used to measure the consistency and change amount. Two objects were measured for each paste, and their maximum diameter and shortest diameter were measured. The average value of a total of four values consisting of the maximum diameter and shortest diameter of the two measurement objects was taken as the consistency.

The consistency of the paste stored at 23°C for 1 day was defined as the initial value, and the differences in consistency from the initial value of the paste stored at 23°C for 14 days and at 50°C for 14 days were calculated, and the value with the larger difference was defined as the change amount. The evaluation criteria are shown below.
AA: The change amount was 0 to 2 mm. It can be determined that the consistency of the paste does not change over time.
A: The change amount was 3 to 4 mm. It can be determined that the consistency of the paste hardly changes over time.
B: The change amount was 5 to 9 mm. It can be determined that the consistency of the paste changes over time.
C: The change amount was 10 mm or more. It can be determined that the consistency of the paste changes significantly over time.

### [Evaluation 4: Sedimentation of paste]

In this evaluation, the paste of 5 g which was filled into an 8 mL cream container and stored at 50 °C for 14 days after preparing the paste was used.

After leaving the paste in a thermostatic room at 23 °C before one hour of conducting the evaluation, the paste in the cream container was stirred with a plastic spatula and the presence or absence of sedimentation of the paste was confirmed. The evaluation criteria are shown below.
A: No sedimentation of the paste was observed. After stirring for 15 seconds, the paste became homogeneous.
B: Sedimentation of the paste was observed. After stirring for 15 seconds, the paste became homogeneous.
C: Sedimentation of the paste was observed. After stirring for 15 seconds, lumps remained and the paste did not become homogeneous.

### [Evaluation 5: Firing property (gloss) at appropriate firing temperature of base material glass]

The firing property (gloss) of the application surface of the fired product prepared in Evaluation 1 were evaluated.
AA: In a state in which gloss was exhibited on the application surface of the fired product and the application surface was uniform.
A: In a state in which gloss was exhibited on the application surface of the fired product but there were mottled application surfaces (brush lines) all over.
B: In a state in which due to insufficient firing, gloss was not exhibited on the application surface of the fired product and there were mottled application surfaces all over.

### [Base material glass]

Glasses G1 to G3 containing SiO₂, Al₂O₃, KzO, NazO, and other components (B₂O₃, LizO, CaO, ZnO or the like) in the ratio shown in Table 1 were prepared by a melting method, and then crushed using a general crusher to prepare base material glasses (a-1) to (a-11). As base material glass (a-12), AS (A shade tone) of Vintage Art Universal (manufactured by SHOFU INC.), which is a commercially available coloring material for dental ceramics, was used. Base material glass (a-12) contains, in addition to the base material glass components, (e) coloring material selected from an inorganic material and (f) fluorescent material selected from an inorganic material, and has 5 µm of the particle diameter D50, 75 µm of the maximum particle diameter D100, and 730 °C of the appropriate firing temperature. Details of each base material glass are as follows.

**[Table 1]**

| Glass | | G1 | G2 | G3 |
|---|---|---|---|---|
| (w/w%) | SiO₂ | 63.3 | 64.4 | 65.5 |
| | Al₂O₃ | 6.6 | 14.5 | 5.5 |
| | K₂O | 4.7 | 7.1 | 9.3 |
| | Na₂O | 6.4 | 12.7 | 4.1 |
| | Other components | 19 (B₂O₃, Li₂O, ZnO) | 1.3 (CaO, MgO) | 15.6 (B₂O₃, Li₂O, CaO) |
| | Total | 100 | 100 | 100 |

### <Preparation of base material glass (a-1)>

Glass G1 was ground using a bead mill crusher until D50 was 0.5 µm.

### <Preparation of base material glass (a-2)>

Glass G1 was ground using a bead mill crusher until D50 was 1.0 µm.

### <Preparation of base material glass (a-3)>

Glass G1 was ground using a bead mill crusher until D50 was 2.0 µm.

### <Preparation of base material glass (a-4)>

Glass G1 was ground using a vibration ball mill crusher until D50 was 4.0 µm.

### <Preparation of base material glass (a-5)>

Glass G1 was ground using a vibration ball mill crusher until D50 was 6.0 µm.

### <Preparation of base material glass (a-6)>

Glass G1 was ground using a vibration ball mill crusher until D50 was 10.0 µm.

### <Preparation of base material glass (a-7)>

Glass G1 was ground using a ball mill crusher until D50 was 15.0 µm.

### <Preparation of base material glass (a-8)>

Glass G1 was ground using a ball mill crusher until D50 was 20.0 µm.

### <Preparation of base material glass (a-9)>

Glass G1 was ground using a ball mill crusher until D50 was 40.0 µm.

### <Preparation of base material glass (a-10)>

Glass G2 was ground using a vibration ball mill crusher until D50 was 6.0 µm.

### <Preparation of base material glass (a-11)>

Glass G3 was subjected to a heat treatment step to precipitate leucite crystals, and then pulverized using a jet mill crusher until D50 became 5.0 µm.

Table 2 shows the test results for each base material glass.

**[Table 2]**

| Base material glass | (a-1) | (a-2) | (a-3) | (a-4) | (a-5) | (a-6) |
|---|---|---|---|---|---|---|
| Average particle diameter D50[µm] | 0.5 | 1 | 2 | 4 | 6 | 10 |
| Maximum particle diameter 100[µm] | 5 | 20 | 45 | 50 | 75 | 100 |
| Presence or absence of crystals | Absence | Absence | Absence | Absence | Absence | Absence |
| Softening point | 560 °C | 560 °C | 560 °C | 560 °C | 560 °C | 560 °C |
| Elution amount | <20 | <20 | <20 | <20 | <20 | <20 |
| Bending strength | >100 | >100 | >100 | >100 | >100 | >100 |
| Thermal expansion coefficient | 8.5 | 8.5 | 8.5 | 8.5 | 8.5 | 8.5 |
| Appropriate firing temperature | 730 °C | 730 °C | 730 °C | 730 °C | 730 °C | 730 °C |

| Base material glass | (a-7) | (a-8) | (a-9) | (a-10) | (a-11) |
|---|---|---|---|---|---|
| Average particle diameter D50[µm] | 15 | 20 | 40 | 6 | 5 |
| Maximum particle diameter 100[µm] | 175 | 200 | 300 | 75 | 25 |
| Presence or absence of crystals | Absence | Absence | Absence | Absence | Leucite |
| Softening point | 560 °C | 560 °C | 560 °C | 605 °C | 640 °C |
| Elution amount | <20 | <20 | <20 | <20 | <20 |
| Bending strength | > 100 | > 100 | > 100 | > 100 | 80 |
| Thermal expansion coefficient | 8.5 | 8.5 | 8.5 | 7 | 14 |
| Appropriate firing temperature | 730 °C | 730 °C | 730 °C | 810 °C | 880 °C |

### [Fine particle silica]

As the fine particle silica, RX50 (average primary particle diameter is 40 nm, manufactured by Nippon Aerosil Co., Ltd.), R974 (average primary particle diameter is 12 nm, manufactured by Nippon Aerosil Co., Ltd.), R812 (average primary particle diameter is 7 nm, manufactured by Nippon Aerosil Co., Ltd.), YA050C (average primary particle diameter is 50 nm, manufactured by Admatechs.), YA100C (average primary particle diameter si 100 nm, manufactured by Admatechs.), and hydrophilic fine particle silica #200 (manufactured by Nippon Aerosil Co., Ltd.) were used.

### [Organic solvent]

Ethanol, propylene glycol, 1,3-butanediol, glycerin, and benzyl benzoate were used as organic solvents.

### [Water]

Water was used as a liquid component other than the organic solvent.

**[Table 3]**

| Organic solvent | Boiling point | Molecular weight | Hydroxy group |
|---|---|---|---|
| 1,3-butanediol | 207 °C | 90.12 | Presence |
| Glycerin | 290 °C | 92.09 | Presence |
| Propylene glycol | 188 °C | 76.09 | Presence |
| Ethanol | 78 °C | 46.07 | Presence |
| Benzyl benzoate | 323 °C | 212.25 | Absence |

### [Inorganic salt]

As inorganic salts, calcium chloride, zinc chloride and calcium nitrate tetrahydrate, which has solubility in any of the above organic solvents, were used.

### [Organic polymer salt]

As the organic polymer salts, sodium polyacrylate (molecular weight 5,000) and sodium carboxymethyl cellulose (molecular weight 700,000), which has solubility in any of the above organic solvents, were used.

**[Table 4]**

| Kind | Component | Example 1 | Example 2 | Example 3 | Example 4 | Example 5 | Example 6 | Example 7 | Example 8 | Example 9 | Example 10 | Example 11 | Example 12 | Example 13 | Example 14 |
|---|---|---|---|---|---|---|---|---|---|---|---|---|---|---|---|
| Base material glass | (a-1) | | | | | | | | | | | | | | |
| | (a-2) | 100.00 | | | | | | | | | | | | | |
| | (a-3) | | 100.00 | | | | | | | | | | | | |
| | (a-4) | | | 100.00 | | | | | | | | | | | |
| | (a-5) | | | | 100.00 | | | | | | | 100.00 | 100.00 | 100.00 | 100.00 |
| | (a-6) | | | | | 100.00 | | | | | | | | | |
| | (a-7) | | | | | | 100.00 | | | | | | | | |
| | (a-8) | | | | | | | 100.00 | | | | | | | |
| | (a-9) | | | | | | | | | | | | | | |
| | (a-10) | | | | | | | | 100.00 | | | | | | |
| | (a-11) | | | | | | | | | 100.00 | | | | | |
| | (a-12) | | | | | | | | | | 100.00 | | | | |
| Silica | RX50 | | | | | | | | | | | 4.84 | | | 2.42 |
| | R974 | 4.84 | 4.84 | 4.84 | 4.84 | 4.84 | 4.84 | 4.84 | 4.84 | 4.84 | 4.84 | | | | 2.42 |
| | R812 | | | | | | | | | | | | 4.84 | | |
| | YA050C | | | | | | | | | | | | | 4.84 | |
| | YA100C | | | | | | | | | | | | | | |
| | #200 | | | | | | | | | | | | | | |
| Organic solvent | 1,3-butanediol | 44.21 | 44.21 | 44.21 | 44.21 | 44.21 | 44.21 | 44.21 | 44.21 | 44.21 | 44.21 | 44.21 | 44.21 | 44.21 | 44.21 |
| | Glycerin | | | | | | | | | | | | | | |
| | Propylene glycol | | | | | | | | | | | | | | |
| | Ethanol | | | | | | | | | | | | | | |
| | Benzyl benzoate | | | | | | | | | | | | | | |
| Inorganic salt | Calcium chloride | 0.15 | 0.15 | 0.15 | 0.15 | 0.15 | 0.15 | 0.15 | 0.15 | 0.15 | 0.15 | 0.15 | 0.15 | 0.15 | 0.15 |
| | Zinc chloride | | | | | | | | | | | | | | |
| | Calcium nitrate tetrahvdrate | | | | | | | | | | | | | | |
| Organic polymer salt | Sodium polyacrylate | | | | | | | | | | | | | | |
| | sodium carboxymethyl cellulose | | | | | | | | | | | | | | |

| Test results | | | | | | | | | | | | | | | |
|---|---|---|---|---|---|---|---|---|---|---|---|---|---|---|---|
| Evaluation 1: Firing property of base material glass at appropriate firing temperature | | A | A | A | A | A | A | A | A | A | A | A | A | A | A |
| Evaluation 2: Presence or absence of bubbles | | A | A | A | A | A | A | A | A | A | A | A | A | A | A |
| Consistency | 23 °C for 1 day [mm] | 20 | 18 | 19 | 19 | 19 | 19 | 19 | 18 | 14 | 18 | 35 | 34 | 34 | 25 |
| | 23 °C for 14 days [mm] | 21 | 19 | 19 | 19 | 19 | 19 | 19 | 18 | 16 | 18 | 35 | 33 | 35 | 26 |
| | 50 °C for 14 days [mm] | 23 | 20 | 20 | 20 | 20 | 21 | 22 | 19 | 16 | 19 | 35 | 36 | 37 | 27 |
| | Change amount | 3 | 2 | 1 | 1 | 1 | 2 | 3 | 1 | 2 | 1 | 0 | 2 | 3 | 2 |
| Evaluation 3: Consistency and change amount of paste | | A | AA | AA | AA | AA | AA | A | AA | AA | AA | AA | AA | A | AA |
| Evaluation 4: Sedimentation of paste | | A | A | A | A | A | A | A | A | A | A | A | A | A | A |
| Evaluation 5 Firing property (gloss) at appropriate firing temperature of base material glass | | AA | AA | AA | AA | AA | AA | AA | AA | AA | AA | AA | AA | AA | AA |

**[Table 5]**

| Kind | Component | Example 15 | Example 16 | Example 17 | Example 18 | Example 19 | Example 20 | Example 21 | Example 22 | Example 23 | Example 24 | Example 25 | Example 26 | Example 27 |
|---|---|---|---|---|---|---|---|---|---|---|---|---|---|---|
| Base material glass | (a-1) | | | | | | | | | | | | | |
| | (a-2) | | | | | | | | | | | | | |
| | (a-3) | | | | | | | | | | | | | |
| | (a-4) | | | | | | | | | | | 30.00 | | |
| | (a-5) | 100.00 | 100.00 | 100.00 | 100.00 | 100.00 | 100.00 | 100.00 | 100.00 | 100.00 | 100.00 | 40.00 | 100.00 | 100.00 |
| | (a-6) | | | | | | | | | | | 30.00 | | |
| | (a-7) | | | | | | | | | | | | | |
| | (a-8) | | | | | | | | | | | | | |
| | (a-9) | | | | | | | | | | | | | |
| | (a-10) | | | | | | | | | | | | | |
| | (a-11) | | | | | | | | | | | | | |
| Silica | RX50 | | | | | | | | | | | | | |
| | R974 | 2.42 | 1.00 | 1.50 | 6.00 | 15.00 | 4.84 | 4.84 | 4.84 | 4.84 | 4.84 | 2.42 | 4.84 | 4.84 |
| | R812 | | | | | | | | | | | 2.42 | | |
| | YA050C | | | | | | | | | | | | | |
| | YA100C | | | | | | | | | | | | | |
| | #200 | 2.42 | | | | | | | | | | | | |
| Organic solvent | 1,3-butanediol | 44.21 | 44.21 | 30.00 | 60.00 | 44.21 | 90.00 | 60.00 | 15.00 | 44.21 | 44.21 | 22.10 | | |
| | Glycerin | | | | | | | | | | | | 44.21 | |
| | Propylene glycol | | | | | | | | | | | 22.10 | | 44.21 |
| | Ethanol | | | | | | | | | | | | | |
| | Benzyl benzoate | | | | | | | | | | | | | |
| Inorganic salt | Calcium chloride | 0.15 | 0.05 | 0.10 | 0.50 | 1.00 | 0.15 | 0.15 | 0.15 | | | 0.10 | 0.15 | 0.15 |
| | Zinc chloride | | | | | | | | | 0.15 | | 0.05 | | |
| | Calcium nitrate tetrahydrate | | | | | | | | | | 0.15 | | | |
| Organic polymer salt | Sodium polyacrylate | | | | | | | | | | | | | |
| | sodium carboxymethyl cellulose | | | | | | | | | | | | | |

| Test results | | | | | | | | | | | | | | |
|---|---|---|---|---|---|---|---|---|---|---|---|---|---|---|
| Evaluation 1: Firing property of base material glass at appropiate firing temperature | | A | A | A | A | A | A | A | A | A | A | A | A | A |
| Evaluation 2: Presence or absence of bubbles | | A | A | A | A | A | A | A | A | A | A | A | A | A |
| Consistency | 23 °C for 1 day [mm] | 25 | 28 | 28 | 22 | 15 | 29 | 24 | 18 | 20 | 20 | 20 | 20 | 21 |
| | 23 °C for 14 days [mm] | 25 | 29 | 29 | 22 | 15 | 30 | 26 | 19 | 20 | 20 | 20 | 20 | 21 |
| | 50 °C for 14 days [mm] | 25 | 32 | 29 | 22 | 15 | 31 | 26 | 20 | 22 | 22 | 21 | 20 | 23 |
| | Change amount | 0 | 4 | 1 | 0 | 0 | 3 | 2 | 2 | 2 | 2 | 1 | 0 | 2 |
| Evaluation 3 Consistency and change amount of paste | | AA | A | AA | AA | AA | A | AA | AA | AA | AA | AA | AA | AA |
| Evaluation 4: Sedimentation of paste | | A | A | A | A | A | A | A | A | A | A | A | A | A |
| Evaluation 5 Firing property (gloss) at appropriate firing temperature of base material glass | | AA | AA | AA | AA | A | AA | AA | AA | AA | AA | AA | AA | AA |

**[Table 6]**

| Kind | Component | Example 28 | Example 29 | Example 30 | Example 31 | Compa rative Example 1 | Compa rative Example 2 | Compa rative Example 3 | Compa rative Example 4 | Compa rative Example 5 | Compa rative Example 6 | Compa rative Example 7 | Compa rative Example 8 | Compa rative Example 9 | Compa rative Example 10 |
|---|---|---|---|---|---|---|---|---|---|---|---|---|---|---|---|
| Base material glass | (a-1) | | | | | 100.00 | | | | | | | | | |
| | (a-2) | | | | | | | | | | | | | | |
| | (a-3) | | | | | | | | | | | | | | |
| | (a-4) | | | | | | | | | | | | | | |
| | (a-5) | 100.00 | 100.00 | 100.00 | 100.00 | | | 100.00 | 100.00 | 100.00 | 100.00 | 100.00 | 100.00 | 100.00 | 100.00 |
| | (a-6) | | | | | | | | | | | | | | |
| | (a-7) | | | | | | | | | | | | | | |
| | (a-8) | | | | | | | | | | | | | | |
| | (a-9) | | | | | | 100.00 | | | | | | | | |
| | (a-10) | | | | | | | | | | | | | | |
| | (a-11) | | | | | | | | | | | | | | |
| Silica | RX50 | | | | | | | | | | | | | | |
| | R974 | 0.80 | 20.00 | 4.84 | 4.84 | 4.84 | 4.84 | | | 4.84 | 4.84 | 4.84 | 4.84 | 4.84 | 4.84 |
| | R812 | | | | | | | | | | | | | | |
| | YA050C | | | | | | | | | | | | | | |
| | YA100C | | | | | | | 4.84 | | | | | | | |
| | #200 | | | | | | | | 4.84 | | | | | | |
| Organic solvent | 1.3-butanediol | 44.21 | 44.21 | 100.00 | 10.00 | 44.21 | 44.21 | 44.21 | 44.21 | 44.21 | | | 44.21 | 44.21 | 22.10 |
| | Glycerin | | | | | | | | | | | | | | |
| | Propylene glycol | | | | | | | | | | | | | | |
| | Ethanol | | | | | | | | | | 44.21 | | | | |
| | Benzyl benzoate | | | | | | | | | | | 44.21 | | | |
| Water | Water | | | | | | | | | | | | | | 22.1 |
| Inorganic salt | Calcium chloride | 0.04 | 2.00 | 0.15 | 0.15 | 0.15 | 0.15 | 0.15 | 0.15 | | 0.15 | 0.15 | | | |
| | Zinc chloride | | | | | | | | | | | | | | |
| | Calcium nitrate tetrahydrate | | | | | | | | | | | | | | |
| Organic polymer salt | Sodium polyacrylate | | | | | | | | | | | | 0.15 | | |
| | sodium carboxymethyl cellulose | | | | | | | | | | | | | 0.15 | |

| Test results | | | | | | | | | | | | | | | |
|---|---|---|---|---|---|---|---|---|---|---|---|---|---|---|---|
| Evaluation 1: Firing property of base material glass at appropiate firing temperature | | A | A | A | A | A | A | A | A | A | A | B | H | H | A |
| Evaluation 2: Presence or absence of bubbles | | A | A | A | A | A | A | A | A | A | A | A | B | B | A |
| Consistency | 23 °C for 1 day [mm] | 25 | 11 | 32 | 8 | 19 | 19 | 25 | 33 | 24 | 19 | 19 | 23 | 25 | 24 |
| | 23 °C for 14 days [mm] | 30 | 11 | 33 | 8 | 24 | 19 | 28 | 34 | 28 | | 19 | 25 | 29 | 29 |
| | 50 °C for 14 days [mm] | 33 | 11 | 37 | 8 | 30 | 19 | 29 | 35 | 35 | | 21 | 25 | 35 | 30 |
| | Change amount | 8 | 0 | 5 | 0 | 11 | 0 | 4 | 2 | 11 | - | 2 | 2 | 10 | 6 |
| Evaluation 3: Consistency and change amount of paste | | H | AA | H | AA | C | AA | A | AA | C | AA | AA | AA | C | H |
| Evaluation 4 Sedimentation of paste | | A | A | A | B | A | C | C | C | A | C | B | B | B | C |
| Evaluation 5 Firing property (gloss) at appropriate firing temperature of base material glass | | AA | H | AA | AA | A | AA | AA | AA | AA | AA | AA | AA | AA | AA |

In Examples 1 to 31, there were no problems in the firing property of the pastes, in change in consistency over time, and in occurrence of sedimentation.

On the other hand, the following problems arose in the Comparative example.

In Comparative Examples 1 and 2, the average particle diameter D50 of the base material glass was not appropriate, therefore large consistency change and sedimentation over time were occurred.

In Comparative Examples 3 and 4, hydrophobic fine particle silica having an inappropriate average primary particle diameter and hydrophilic fine particle silica were used, therefore sedimentation over time were occurred.

In Comparative Examples 5 and 10, no inorganic salt was contained, therefore a large change in consistency occurred.

In Comparative Examples 6 and 7, only ethanol or only benzyl benzoate was used as the organic solvent, therefore sedimentation over time and black discoloration of the application surface occurred.

In Comparative Examples 8 and 9, an organic polymer component was used, therefore black discoloration and bubbles occurred on the application surface.

From the above results, the dental porcelain paste of the present invention is a paste that maintains a constant paste property even during long-term storage, and suppress black discoloration due to carbonization and cloudiness due to bubbles during firing even in the case of containing organic components, and therefore significantly improves the problems of the conventional technology.

With respect to the use of substantially any plural and/or singular terms herein, those having skill in the art can translate from the plural to the singular and/or from the singular to the plural as is appropriate to the context.

Although the description herein has been given with reference to the drawings and embodiments, it should be noted that those skilled in the art may make various changes and modifications on the basis of this invention without difficulty. Accordingly, any such changes and modifications are intended to be included in the scope of the embodiments.

### [Industrial applicability]

The dental porcelain paste provided by the present invention has storage stability that can maintain a constant paste property for a long-term, and are excellent in application property and aesthetic property. Therefore, it can be applied to various dental crown restorations in restorative treatment in the dental field.

## Claims

1. A dental porcelain paste for preparing a dental prosthesis device, comprising
(a) base material glass having an average particle diameter D50 of 1 to 20 µm,
(b) hydrophobized fine particle silica having an average primary particle diameter of 1 to 50 nm,
(c) organic solvent having a boiling point of 100 to 300°C, and
(d) inorganic salt.

2. The dental porcelain paste according to claim 1, wherein
the dental porcelain paste comprises, with respect to 100 parts by mass of the (a) base material glass,
1 to 15 parts by mass of the (b) hydrophobized fine particle silica,
15 to 90 parts by mass of the (c) organic solvent, and
0.05 to 1.0 parts by mass of the (d) inorganic salt.

3. The dental porcelain paste according to claim 1, wherein
the dental porcelain paste further comprises (e) coloring material and/or (f) fluorescent material which are inorganic materials.

4. The dental porcelain paste according to claim 2, wherein
the dental porcelain paste further comprises (e) coloring material and/or (f) fluorescent material which are inorganic materials.

5. The dental porcelain paste according to any one of claims 1 to 4, wherein
a maximum particle diameter D100 of the (a) base material glass is 200 µm or less.
